# FASCICULE DE BREVET EUROPEEN

(11) **EP 0 875 293 B1**
(45) Date de publication et mention de la délivrance du brevet: **18.06.2003**
(21) Numéro de dépôt: 98420075.8
(22) Date de dépôt: 28.04.1998
(51) Int. Cl.: B01L 11/00, G01N 33/49

(54) **Dispositif de transfert de gouttes calibrées d'un liquide contenu dans un segment de tuyauterie en matière plastique de faible diamètre fermé à ses extrémités**
Vorrichtung zur Tropfenübertragung einer Flüssigkeit, die zwischen den beiden abgedichten Extremitäten eines Kunststoffschlauchs von geringem Durchmesser beinhaltet ist
Device for the transfer of calibrated drops of a liquid contained in a low diameter plastic segment tube closed at both ends

(30) Priorité: 28.04.1997 FR 9705335
(43) Date de publication de la demande: 04.11.1998
(73) Titulaire: Pharmabio S.à.r.l., 78100 St Germain en Laye (FR)
(72) Inventeur: Depaulis, Robert, 78112 Fourquex (FR)
(74) Mandataire: Schmitt, John

(56) Documents cités:
- EP-A- 0 350 792
- WO-A-94/20216
- BE-A- 904 832
- US-A- 4 176 451
- US-A- 4 790 842
- US-A- 5 254 312

## Description

L'invention concerne un dispositif transfert de gouttes calibrées de liquide emprisonné dans un segment de tuyauterie en matière plastique souple de faible diamètre.

Lorsqu'on prélève une poche de sang sur un donneur, le sang est amené depuis l'aiguille de prélèvement jusqu'à la poche de stockage par une tuyauterie souple ; à la fin du prélèvement la tuyauterie qui est restée pleine de sang est divisée en plusieurs compartiments étanches par écrasement de la paroi de la tuyauterie par des électrodes chaudes qu'on appellera ci-après « segments » ; lors de l'utilisation de la poche, pour effectuer une transfusion, il est nécessaire de contrôler préalablement à cette dernière la compatibilité du sang qu'elle contient avec le sang du receveur ; pour effectuer cette opération on prélève un segment sur la poche, on coupe une extrémité du tube avec un outil coupant, et on presse le segment pour en extraire une goutte ou plusieurs gouttes que l'on mélange chacune avec un produit réactif ; cette opération est réalisée souvent dans la précipitation et le stress et dans un environnement difficile où il est facile de se blesser avec des outils coupants. Les problèmes de contamination du personnel soignant, consécutifs à des blessures qu'ils se sont faites et qui ont été souillées par du sang de provenance extérieure, conduit à rechercher comment sécuriser l'opération de transfert du sang depuis le segment pour le mettre en contact avec un produit réactif.

Il existe sur le marché un dispositif qui a la forme générale d'une éprouvette dont l'ouverture est occupée, d'une part par un entonnoir, et d'autre part par un dispositif de versage ; l'entonnoir est prolongé d'un cylindre de guidage à l'extrémité duquel est placée coaxialement une aiguille creuse ; lorsqu'on enfonce l'extrémité du segment dans l'entonnoir puis dans le tube de guidage, son extrémité vient en contact avec l'aiguille creuse qui perfore la paroi du tube ; lorsqu'on presse le segment on transfère le sang dans l'éprouvette où il est dilué par agitation manuelle de l'éprouvette ; ensuite le sang dilué est évacué, par simple gravité par le dispositif de versage qui comporte notamment un tube calibré de manière à contrôler le volume de la goutte de sang dilué ; ce dispositif peut être directement utilisé dans une centrifugeuse. L'avantage essentiel de ce dispositif est que l'aiguille est totalement hors de portée du manipulateur ; cela implique que sa fabrication soit réalisée par l'assemblage étanche de plusieurs pièces plastiques qui ne peut être obtenu que par une reprise et des soudures qui doivent être contrôlées ; la polyvalence de ce dispositif est destinée à justifier son coût ; en fait il est trop complexe à manipuler et peu fiable lorsqu'il s'agit de tester seulement une poche de sang ; notamment la présence de diluant, qui est rendu obligatoire pour augmenter la fluidité du mélange afin de pouvoir ensuite le verser, ne permet pas de savoir la quantité effective de sang qu'il y a dans la goutte extraite de l'éprouvette. Un deuxième dispositif qui vient d'apparaître sur le marché est constitué d'un tube ouvert à une extrémité et fermé à l'autre, cette dernière extrémité étant traversée par une canule placée dans l'axe de symétrie du tube, comme cela est réalisé dans le procédé « VACUTAINER » qui est utilisé pour aspirer du sang dans un flacon sous vide, avec une différence que la canule ne fait qu'affleurer à l'extérieur du côté fermé du tube et ne comporte pas une extrémité de canule pointue permettant de piquer dans la veine ou dans une dérivation du tube de remplissage d'une poche de sang ; on introduit le segment par son extrémité dans le tube, en faisant glisser le tube entre quatre rails de guidage en excroissance à l'intérieur de la cavité du tube, jusqu'à ce qu'il vienne se perforer sur la pointe interne de la canule. Un des problèmes d'un tel dispositif est qu'il manque de fiabilité lors de la perforation ; en effet, on introduit d'abord dans le tube l'extrémité soudée du segment qui se trouve placée en principe dans la zone de l'axe du tube et c'est donc elle qui a toutes les chances d'entrer en contact en premier avec la pointe de la canule et offrir une résistance significative à la perforation ; comme on a dégagé l'espace autour des quatre rails de guidage pour permettre à la soudure de l'extrémité du segment d'entrer sans difficultés dans le tube, le segment peut se déformer sous l'action de la poussée supplémentaire due au blocage de son extrémité soudée sur la pointe de la canule en fléchissant et en glissant par déformation entre deux rails de guidages consécutifs, ce qui peut entraîner la torsion de la canule et sa destruction ; enfin le fait que la canule affleure à l'extérieur rend plus difficile l'introduction du sang dans un orifice de petit diamètre et ne permet pas d'obturer facilement la canule, pour éviter que le sang ne soit projeté à l'extérieur de la canule au moment de la perforation si le segment est sous pression, et pour éviter les souillures de sang lors du stockage après usage.

L'objet de l'invention est de proposer un dispositif de transfert fiable permettant de transférer une ou plusieurs gouttes de sang depuis un segment jusqu'au contact d'un produit réactif dans des conditions acceptables de sécurité pour le manipulateur.

Sur les dessins annexés :
La figure 1 représente une coupe longitudinale du dispositif de transfert selon l'invention.
La figure 2 représente le dispositif de transfert de la figure 1 vu du côté ouvert.
La figure 3 représente le dispositif de transfert de la figure 1 avec un segment en place et le capuchon en place après le prélèvement de sang.
La figure 4 représente une coupe transversale du segment lorsqu'il est plié en deux.
La figure 5 représente une vue en élévation du dispositif de transfert de la figure 1 prêt à l'emploi.

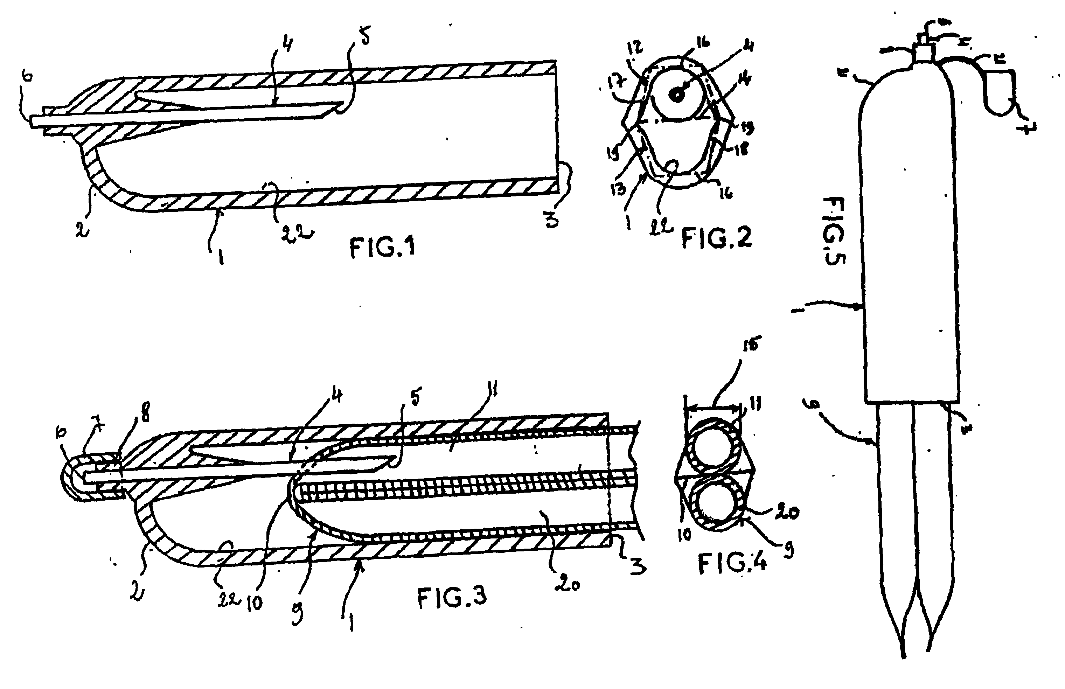

Le dispositif de transfert objet de l'invention consiste en un tube 1 (figure 1), ayant une extrémité ouverte 3 et une extrémité fermée 2, dont l'extrémité fermée 2 est traversée par une canule 4 de préférence métallique placée parallèlement aux génératrices du tube 1 ; l'extrémité intérieure 5 de la canule 4 intérieure au tube 1, est d'une longueur telle qu'elle est inaccessible de l'extérieur par le manipulateur, et elle comporte une pointe ; l'extrémité extérieure 6 de la canule 4, qui est généralement extérieure au tube 1, est au contraire conçue pour éviter les blessures ; l'extrémité extérieure 6 de la canule est, dans une version préférée de l'invention, simplement coupée perpendiculairement à la direction générale de la canule 4 et susceptible d'être refermée après usage par un capuchon 7 (figure 3) qui vient s'emboîter directement sur l'extrémité extérieure ou bien sur une embase 8 prévue à cet effet à l'extrémité fermée 2 du tube 1 ; pour améliorer la sécurité vis-à-vis de l'extrémité extérieure 6 (figure 1), qui peut néanmoins provoquer une blessure en cas de choc violent, on fait en sorte que l'extrémité extérieure 6 de la canule 4 ne dépasse que de un ou deux millimètres d'une embase 8 présentant une surface apparente suffisante pour absorber l'essentiel des efforts sur la peau de l'opérateur résultant d'un choc violent. Le tube 1 est de préférence en matière plastique injectée ; le profil interne 22 de la section du tube 1 (figure 2) est déterminée de manière qu'on puisse y introduire un segment 9 (figure 3 et figure 4) préalablement plié en deux, en y faisant pénétrer, en tête, l'extrémité du segment 9 comportant la pliure 10, et suivant une orientation contrôlée de manière que la canule 4 puisse pénétrer à coup sûr dans l'un des demi-segments 11 ;en effet, la pliure 10 est alors placée dans le plan tangent commun aux deux demi-segments et passant par leur génératrice de contact et donc dans une position parfaitement contrôlée, hors de l'espace occupé par la section circulaire de chacun des demi-segments ; le pliage du segment en augmente sa rigidité, par le fait qu'il y a deux demi-segments associés côte à côte, qui est facilitée par la mise sous pression du liquide contenu qui tend la paroi et facilite la pénétration de la canule ; dans ces conditions, il est possible d'éviter les projections en positionnant le capuchon 7 sur l'extrémité 6 de la canule 4 avant d'introduire le segment 9 replié ; le profil interne 22 de la section du tube 1 (figure 2) s'inscrit dans un ensemble formé de deux trapèzes isocèles 12 et 13 égaux opposés l'un à l'autre par leur grande base 14 dont la longueur est sensiblement égale à une fois et demi le diamètre 15 (figure 4) du segment 9 et la petite base 16 (figure 2) ainsi que la hauteur des trapèzes 12 et 13 étant sensiblement égale au diamètre 15 (figure 4) du segment 9 ; de préférence les parties du profil interne 22 tangentes aux petites bases 16 sont arrondies pour épouser au mieux la forme des demi-segments ; on peut donner aux parties du profil interne 22 qui rejoignent les parties arrondies entre elles, une forme de deux accolades 17 et 18 en opposition dont la pointe 19 est tournée vers l'extérieur ; la largeur du profil interne 22 au niveau des pointes 19 des accolades est sensiblement égale à la largeur du segment au niveau de sa pliure 10 qui est égale à son demi périmètre et qui est égale à la longueur de la grande base 14 des trapèzes 12 et 13. Lorsqu'on introduit le segment 9 (figure 3) replié dans le tube 1 les deux demi-segments 11 et 20 du segment 9 sont superposées et occupent sensiblement chacun la moitié de la section du tube 1 ; pour être sûr qu'en enfonçant le segment 9 plié en deux la canule 4 va perforer la paroi d'un demi-segment 11 sans toutefois ressortir de l'autre côté, on place la canule 4 (figure 4) au centre de gravité de la surface circonscrite par le trapèze 12 occupée par le demi-segment 11 (figure 3) ; dans ces conditions, lorsque la canule 4 a perforé la paroi du demi-segment 11 correspondant, elle se trouve dans l'axe de ce demi-segment 11, qui est guidé linéairement au cours de l'enfoncement par le profil interne 22 de la section du tube 1 (figure 2) et par l'autre demi-segment 20, et elle ne peut en ressortir. La longueur de la canule 4 à l'intérieur du tube est déterminée pour, tout en restant inaccessible de l'extérieur, qu'on puisse y introduire un segment 9 de faible longueur ; à titre d'exemple non limitatif la longueur intérieure du tube 1 peut être de l'ordre de quatre à sept centimètres pour des segments 9 d'un diamètre 15 (figure 4) de quatre à cinq millimètres, tandis que l'extrémité intérieure 5 pointue de la canule 4 se trouve à une distance allant de quinze à vingt cinq millimètres de l'extrémité ouverte 3 du tube 1.

Lorsque le segment 9 (figure 5) replié est en place dans le tube 1, il suffit de presser sur le segment 9 pour que le sang s'échappe goutte à goutte de l'extrémité extérieure 6 de la canule 4 ; il est possible de laisser le segment 9 enfoncé dans le tube 1 après usage et de le stocker pour un contrôle ultérieur ; dans le cadre d'un contrôle ultérieur on peut prendre deux segments consécutifs liés entre eux par une soudure écrasant le tube, et replier les deux segments l'un contre l'autre pour introduire en tête la soudure dans le tube 1 de manière qu'un seul des deux segments soit ouvert tandis que l'autre reste intact. On peut utiliser ce dispositif de transfert pour des segments contenant d'autres produits liquides ou pâteux tels que du sang tout en restant dans le domaine de l'invention ; il permet, par exemple sans que cet exemple soit limitatif, d'appliquer des colles avec précision.

La canule 4 (figure 1) du dispositif de transfert n'étant pas nécessairement stérile, elle est dans une version préférée de l'invention placée dans le moule d'injection du tube 1 qui vient se mouler autour ; on peut mouler en même temps le capuchon 7 (figure 3 et figure 5) qui viendra fermer l'extrémité extérieure 6 de la canule 4 après usage pour éviter que le sang ne continue à se répandre, et éventuellement relier le capuchon 7 au tube 1 par une tige souple 21 venant d'injection et qui peut être constituée par le canal d'alimentation du capuchon 7 en matière plastique.

## Revendications

1. Dispositif de transfert d'un liquide contenu dans un segment (9) tubulaire en matière plastique fermé par soudure à ses extrémités, par introduction d'une canule (4) dans ledit segment, dispositif constitué, d'une part d'un tube (1) ayant une extrémité ouverte (3) et une extrémité fermée (2) qui est traversée, parallèlement aux génératrices du tube (1), par la canule (4) dont l'extrémité intérieure (5) au tube (1) est pointue et, d'autre part, du segment (9) introduit dans le tube 1, **caractérisé en ce que** le segment (9) est introduit plié en deux suivant une pliure (10) de manière que les demi segments (11) et (20) soient superposés et de manière que la pliure (10) soit placée en avant dans le tube (1) qui comporte des moyens de guidage, la canule (4) étant placée par rapport au profil interne (22) du tube (1) pour pénétrer dans l'axe du demi-segment (11) du segment (9), et **en ce que** l'extrémité extérieure (6) de la canule (4) comporte des moyens de protection qui lui sont associés.

2. Dispositif de transfert de liquide suivant la revendication 1, **caractérisé en ce que** les moyens de guidage. sont assurés par la forme du profil interne (22) de la section du tube (1) qui s'inscrit dans un ensemble formé de deux trapèzes isocèles (12) et (13) égaux opposés l'un à l'autre par leur grande base (14) dont la longueur est sensiblement égale à une fois et demi le diamètre (15) du segment (9) et la petite base (16) ainsi que la hauteur des trapèzes (12) et (13) est sensiblement égale au diamètre (15) du segment (9).

3. Dispositif de transfert de liquide suivant la revendication 2, **caractérisé en ce que** les parties du profil interne (22) tangentes aux petites bases (16) sont arrondies et reliées entre elles par deux accolades (17) et (18) en opposition dont la pointe (19) est tournée vers l'extérieur.

4. Dispositif de transfert de liquide suivant la revendication 3, **caractérisé en ce que** les pointes (19) des accolades (17) et (18) sont distantes d'environ une fois et demi le diamètre (15) du segment (9).

5. Dispositif de transfert de liquide suivant la revendication 2, **caractérisé en ce que** la canule (4) est positionnée, par rapport au profil interne (22), au centre de gravité de la surface circonscrite par le trapèze (12) occupée par le demi-segment (11).

6. Dispositif de transfert suivant la revendication 1, **caractérisé en ce que** l'extrémité extérieure (6) de la canule (4) dépasse de l'extrémité fermée (2) du tube (1).

7. Dispositif de transfert de liquide suivant l'une quelconque des revendications 1 et 6, **caractérisé en ce que** l'extrémité extérieure (6) de la canule (4) est susceptible d'être refermée après usage par un capuchon (7) qui vient s'emboîter directement sur l'extrémité extérieure (6) et/ou sur une embase (8).

8. Dispositif de transfert de liquide suivant les revendications 6 et 7, **caractérisé en ce que** l'extrémité extérieure (6) de la canule (4) ne dépasse que d'un ou deux millimètres de l'embase (8).

9. Dispositif de transfert de liquide suivant la revendication 7, **caractérisé en ce que** le capuchon (7) est moulé en même temps que le tube (1) et qu'il est relié au tube (1) par une tige souple (21) provenant directement d'injection.

## Patentansprüche

1. Transfervorrichtung aus Plastik einer in einem röhrenförmigen, an seinen Enden durch Verschweißen geschlossenen Segment (9) enthaltenen Flüssigkeit durch Einführen einer Kanüle (4) in das besagte Segment, wobei die Vorrichtung einerseits aus einer Röhre (1) gebildet wird, die ein offenes Ende (3) und ein geschlossenes Ende (2) hat, das parallel zu den Mantellinien der Röhre (1) durch die Kanüle (4) durchquert wird, deren inneres Ende (5) an der Röhre (1) zugespitzt ist, und andererseits aus dem in die Röhre 1 eingeführten Segment (9), **dadurch gekennzeichnet, dass** das Segment (9) gemäß eines Knicks (10) in zwei Teile geknickt derart eingeführt wird, dass die halben Segmente (11) und (20) derart übereinander gelagert werden, dass der Knick (10) in der Röhre (1) nach vorne platziert wird, die Führungsmittel enthält, wobei die Kanüle (4) im Verhältnis zum inneren Profil (22) der Röhre (1) platziert wird, um in die Achse des halben Segments (11) des Segments (9) einzudringen, und dass das äußere Ende (6) der Kanüle (4) Schutzmittel enthält, die ihm zugeordnet sind.

2. Transfervorrichtung für Flüssigkeit gemäß Anspruch 1, **dadurch gekennzeichnet, dass** die Führungsmittel durch die Form des inneren Profils (22) des Durchmessers der Röhre (1) gewährleistet sind, die eine aus zwei gleichschenkligen, gleichen, einander gegenüber liegenden Trapezen (12) und (13) durch ihre große Basis (14), deren Länge deutlich gleich eineinhalb mal der Durchmesser (15) des Segments ist, und die kleine Basis (16) sowie der Höhe der Trapeze (12) und (13) gebildeten Einheit bildet und deutlich gleich dem Durchmesser (15) des Segments (9) ist.

3. Transfervorrichtung für Flüssigkeit gemäß Anspruch 2, **dadurch gekennzeichnet, dass** die den kleinen Basen (16) anliegenden Teile des inneren Profils (22) abgerundet und untereinander durch zwei Bögen (17) und (18) gegenüber verbunden sind, deren Spitze (19) nach außen gerichtet ist.

4. Transfervorrichtung für Flüssigkeit gemäß Anspruch 3, **dadurch gekennzeichnet, dass** die Spitzen (19) der Bögen (17) und (18) ungefähr eineinhalb mal der Durchmesser (15) des Segments (9) voneinander entfernt sind.

5. Transfervorrichtung für Flüssigkeit gemäß Anspruch 2, **dadurch gekennzeichnet, dass** die Kanüle (4) im Verhältnis zum inneren Profil (22) im Schwerkraftzentrum der vom Trapez. (12) umschriebenen, vom halben Segment (11) eingenommenen Fläche positioniert ist.

6. Transfervorrichtung gemäß Anspruch 1, **dadurch gekennzeichnet, dass** das äußere Ende (6) der Kanüle (4) über das geschlossene Ende (2) der Röhre (1) herausragt.

7. Transfervorrichtung für Flüssigkeit gemäß Anspruch 1 bis 6, **dadurch gekennzeichnet, dass** das äußere Ende (6) der Kanüle (4) geeignet ist, nach Gebrauch durch eine Kappe (7) verschlossen zu werden, die direkt auf das äußere Ende (6) und / oder auf eine Fußfläche (8) passt.

8. Transfervorrichtung für Flüssigkeit gemäß Anspruch 6 und 7, **dadurch gekennzeichnet, dass** das äußere Ende (6) der Kanüle (4) nur um einen oder zwei Millimeter aus der Fußfläche (8) herausragt.

9. Transfervorrichtung für Flüssigkeit gemäß Anspruch 7, **dadurch gekennzeichnet, dass** die Kappe (7) zur selben Zeit wie die Röhre (1) abgeformt wird und dass sie durch einen direkt aus dem Einspritzen stammenden flexiblen Stift (21) mit der Röhre (1) verbunden ist.

## Claims

1. Device for transferring a liquid contained in a plastic tubular segment (9) weld-seamed at its extremities by introducing a hollow needle (4) into said segment, said device being constituted firstly by a tube (1) with an open extremity (3) and a closed extremity (2) which is traversed parallel to the generating lines of the tube (4) by the hollow needle (1), whose one extremity (5) inside the tube (1) is pointed, and secondly a segment (9) introduced in the tube (1), **characterised in that** the segment (9) is introduced folded into two along a fold (10) so that the half-segments (11) and (20) are superimposed and so that the fold (10) is placed in front in the tube (1) which comprises guiding means, the hollow needle (4) being placed with respect to the internal profile (22) of the tube (1) so as to penetrate into the axis of the half-segment (11) of the segment (9), and **in that** the external extremity (6) of the hollow needle (4) comprises protection means associated with it.

2. Liquid transfer device according to claim 1, **characterised in that** the guiding means are provided by the shape of the internal profile (22) of the section of the tube (1) which fits inside a unit formed of two equal isosceles trapeziums (12) and (13) opposite each other via their base (14) whose length is approximately equal to one and a half times the diameter (15 ) of the segment (9) and the small base (16), and **in that** the height of the trapeziums (12) and (13) is approximately equal to the diameter of the segment (9).

3. Liquid transfer device according to claim 2, **characterised in that** the portions of the internal profile (22) tangent to the small bases (16) are rounded and interconnected by two opposing accolades (17) and (18) whose point (19) faces outwards.

4. Liquid transfer device according to claim 3, **characterised in that** the points (19) of the accolades (17) and (18) are distant from each other by about one and a half times the diameter (15) of the segment (9).

5. Liquid transfer device according to claim 2, **characterised in that** the hollow needle (4) is located, with respect to the internal profile (22), at the centre of gravity of the surface circumscribed by the trapezium (12) occupied by the half-segment (11).

6. Liquid transfer device according to claim 1, **characterised in that** the external extremity (6) of the hollow needle (4) extends beyond the closed extremity (2) of the tube (1).

7. Liquid transfer device according to claim 1 or 6, **characterised in that** the external extremity (6) of the hollow needle (4) is able to be closed again after being used by a cap (7) which is directly nested on the external extremity (6) and/or on a base (8).

8. Liquid transfer device according to claims 6 and 7, **characterised in that** the external extremity (6) of the hollow needle (4) exceeds the base (8) by only one or two millimetres.

9. Liquid transfer device according to claim 7, **characterised in that** the cap (7) is moulded at the time as the tube (1) and that it is connected to the tube (1) by a flexible rod (21) originating directly from injection.
